# EUROPEAN PATENT APPLICATION

(11) **EP 1 245 280 A2**
(43) Date of publication of application: **02.10.2002**
(21) Application number: 02007456.3
(22) Date of filing: 30.03.2002
(51) Int. Cl.: B01L 3/00, G01N 1/00, G01N 33/483

(54) **Adaptor for use with point-of-care testing cartridge**

(30) Priority: 30.03.2001 US 280432 P
(71) Applicant: Becton Dickinson and Company, Franklin Lakes, New Jersey 07417-1880 (US)
(72) Inventor: Francavilla, Frank, Newton, New Jersey 07860 (US); Maclean Crawford, Jamieson William, New York, New York (US)
(74) Representative: von Kreisler, Alek, Dipl.-Chem.

(57) **Abstract**

An adaptor (10) is provided to facilitate delivery of a fluid specimen from a syringe to a point-of-care testing cartridge. The adaptor (10) includes opposed proximal (70) and distal ends (72) and a passage (74) extending between the ends. The proximal (70) end of the adaptor (10) defines a Luer fitting (75) configured for mating with a Luer tip of a syringe. The distal end (72) of the adaptor defines a small diameter nose (84) configured for insertion into the entry port of a testing cartridge. An outlet passage (82) extends through the nose (84) and is dimensioned to create small droplets of liquid that can be delivered easily to the testing cartridge. Radially aligned fins (92) are disposed proximally of the nose (84) and define channels between the fins for enabling venting of air from the testing cartridge.

## Description

### RELATED APPLICATIONS

This application claims priority on U.S. Provisional Patent Appl. No. 60/280,432 filed on March 30, 2001.

### BACKGROUND OF THE INVENTION

### 1. Field of the Invention

The subject invention relates to an adaptor mounted to a syringe to facilitate alignment of the syringe with the entry port to a point-of-care testing cartridge and to facilitate transfer of a fluid specimen from a syringe.

### 2. Description of the Related Art

Many medical procedures require diagnostic tests to be performed on a sample of a patient's fluid. Fluid often is collected from a patient by employing a needle holder assembly and one or more evacuated tubes. Fluid also can be collected in a syringe. A syringe may be used with a metallic needle to obtain a fluid sample from a patient. However, syringes often are connected directly to an established arterial or venous line to obtain a fluid sample. The fluid collected in the syringe then may be transferred to a tube. The tubes are labeled carefully and shipped to a laboratory for analysis. The results of the laboratory analysis then are reported back to the health care provider. The results, of course, could be rushed in emergency situations, but absent an emergency would require more then one day between the time the sample is drawn from the patient to the time that the laboratory analysis is reported to the health care provider.

Devices have been developed for performing at least certain diagnostic tests on a sample of fluid at the point-of-care. The point-of-care diagnostic equipment includes a syringe for receiving a sample of fluid from a patient, a small disposable testing cartridge for receiving a portion of the fluid from the syringe and a portable clinical analyzer for analyzing the fluid and outputting the results. Combinations of testing cartridges and portable clinical analyzers are marketed in the United States by i-STAT Corporation, AVL Scientific Corporation and Diametrics Medical, Inc. The systems produced by these and other companies share certain common features. In particular, the testing cartridge of each system typically has a small rectangular housing about 1" x 2" and about .25" thick. The housing includes an internal reservoir with a volume of between about 40µl and 125µl. An inlet port extends through an external wall of the testing cartridge and communicates with the internal reservoir. The cartridge further includes contact pads and sensors that can be placed in communication with the portable clinical analyzer. An example of an i-STAT point-of-care testing cartridge is shown in U.S. Patent No. 5,638,828.

The prior art point-of-care testing systems are employed with a syringe that is used to draw a sample of fluid from a patient. The syringe then may be used to eject a portion of the fluid sample into the inlet port of the point-of-care testing cartridge. However, some testing cartridges are operative to automatically draw fluid from the syringe. The inlet port of the cartridge then is closed and the cartridge is placed in communication with the portable clinical analyzer for performing certain specified diagnostic tests on the sample of fluid in the cartridge. The analyzer then provides a very quick output of the test results without the need for sending the fluid sample to the laboratory.

Point-of-care testing systems provide several efficiencies over systems that require virtually all diagnostic tests to be performed at a location remote from the point-of-care. The small size of the testing cartridge facilitates storage and shipment of the cartridges while also contributing to the portability of the system. However, with regards to transferring a collected sample to the cartridge, the small cartridges can be very difficult to use. For example, alignment of the distal end of the syringe with the inlet port of the testing cartridge can be complicated and difficult. A misalignment or imprecise mating of the syringe with the inlet port of the testing cartridge can lead to a loss of a portion of the collected fluid sample. Additionally, it is difficult to use a syringe for accurately dispensing the proper volume of liquid. Too small a volume may prevent proper testing by the cartridge and the associated portable clinical analyzer. Too large a volume can cause splattering or spillage. Similarly an overfill can result in splatter when the cover of the point-of-care testing cartridge is closed. Fluid that is not delivered efficiently from the syringe into the inlet port of the testing cartridge create the potential for disease transmission. Similarly, a loss of fluid during the transfer from the syringe to the testing cartridge can leave an insufficient volume of fluid for performing the required diagnostic tests. An insufficient volume of fluid to perform the required tests can require the health care worker to return to the patient for a second sample of fluid. This is time consuming for the health care worker and traumatic for the patient. Additionally, some testing cartridges may require an insufficiently filled cartridge to be discarded and a new cartridge to be employed with the new sample of fluid. Thus, inefficiencies in the transfer of fluid from the syringe to the testing cartridge can generate excess costs for additional testing cartridges.

The direct transfer of fluid from a syringe to a testing cartridge can cause the syringe tip to close off the entry port and prevent venting of air from the testing cartridge. Thus bubbles are created. Bubbles reduce the volume of fluid and can affect test results

### SUMMARY OF THE INVENTION

The subject invention is directed to a locator adaptor for use with a point-of-care testing cartridge and with a syringe assembly. The point-of-care testing cartridge may be a prior art testing cartridge as described above, or any yet-to-be developed testing cartridge for performing point-of-care diagnostic analysis on a collected specimen of blood or other bodily fluid. The testing cartridge comprises a housing having an internal reservoir for receiving a specimen to be tested. The housing may be substantially rectangular, with opposed top and bottom walls and a plurality of side walls. An entry port extends through the top wall and that communicates with the internal reservoir of the testing cartridge. The testing cartridge may further include contact pads and sensors that can be placed in communication with a portable clinical analyzer for performing point-of-care analysis of the collected specimen.

The syringe assembly that is used with the snap on adaptor may be a conventional prior art syringe assembly. The syringe assembly includes a body with opposed proximal and distal ends. A barrel extends distally from the proximal end of the body and defines a fluid receiving chamber that is widely open at the proximal end. A Luer tip projects from the barrel to the distal end of the syringe body and includes a passage that communicates with the fluid receiving chamber. The Luer tip includes a conically tapered outer surface that is dimensioned and configured for mating with the tapered proximal entry to the hub of a needle assembly or with the base of a blunt plastic cannula. The distal end of the syringe body may further have an internally threaded Luer collar that projects from the distal end of the barrel and concentrically around the Luer tip. The threads of the Luer collar can be threadedly engaged with projections at the proximal end of the hub of a needle assembly or with comparable projections at the proximal end of a blunt plastic cannula. Luer tips, Luer collars and mating structures on needles or cannulas are known in the art.

The syringe assembly further includes a plunger that is slidably received in the open proximal end of the fluid receiving chamber defined by the syringe barrel. Distal movement of the plunger in the fluid receiving chamber will expel a fluid from the chamber and through the Luer tip. Proximal movement of the plunger in the chamber will draw fluid through the Luer tip and into the chamber.

The syringe assembly with which the walled adaptor is used may further include a needle assembly, a plastic Luer fitting or a blunt plastic cannula for accessing blood or other bodily fluid to be tested. A conventional prior art needle assembly includes an elongate metallic needle cannula having a proximal end, a pointed distal end and a lumen extending between the ends. The prior art needle assembly further includes the plastic hub having opposed proximal and distal ends. The distal end of the hub is securely mounted to the proximal end of the needle cannula. The proximal end of the hub is configured for fluid-tight engagement with the Luer tip. Additionally, the proximal end of the hub may include lugs for threaded engagement with the internal threads on a Luer collar that may be present on the syringe. A plastic Luer fitting or blunt plastic cannula typically is unitarily molded from a plastic material and has opposite proximal and distal ends and a lumen extending between the ends. The proximal end of the plastic Luer fitting blunt plastic cannula may have the same shape as the proximal end of the hub for the above-described needle assembly. The distal end of the blunt plastic cannula may be tapered sufficiently to pierce a septum across a fitting on an IV access system or blood collection set.

The locator adaptor of the subject invention may be unitarily molded from a plastic material. More particularly, the locator adaptor is substantially tubular with opposed proximal and distal ends and a passage extending therebetween. Portions of the passage adjacent the proximal end of the locator adaptor are substantially conically generated and are dimensioned for fluid-tight engagement over the Luer tip of a conventional prior art syringe. Exterior portions of the locator adaptor adjacent the proximal end may be provided with a pair of diametrically opposite lugs for threaded engagement with threads of a Luer collar on a prior art syringe.

The distal end of the locator adaptor includes a small diameter nose through which the passage of the locator adaptor extends. Portions of the passage in the nose of the locator adaptor are much smaller than cross-sectional dimensions of the passage adjacent the proximal end of the adaptor. More particularly, the cross-sectional dimensions of the passage through the nose of the adaptor are sufficiently small to control the volume of transferred fluid precisely and to produce droplets of a specimen flowing through the adaptor that are sufficiently small to prevent overfill or spillage of the specimen adjacent the entry port to the testing cartridge. The external cross-sectional dimensions of the nose of the adaptor also are much smaller than the external cross-sectional dimensions adjacent the proximal end of the locator adaptor. More particularly, the exterior of the nose of the locator adaptor is dimensioned cross-sectionally for easy insertion into the entry port of the testing cartridge. The length of the nose is sufficiently long to facilitate visual alignment of the nose with the entry port. However, the nose is not so long as to contact interior surfaces of the testing cartridge directly opposite the entry port in a way that would impede fluid flow through the nose.

The locator adaptor further includes a plurality of radially aligned fins extending outwardly substantially adjacent the nose of the locator adaptor. The fins may be stepped to define smaller dimensions closer to the nose and larger dimensions at locations spaced from the nose. The stepped configuration facilitates visual alignment of the locator adaptor with the entry port of the testing cartridge. The fins also provide channels between adjacent fins to facilitate venting of air adjacent the entry port to the testing cartridge. The ability to vent air contributes to a smooth flow of blood or other liquid specimen from the syringe to the reservoir of the testing cartridge. Furthermore, efficient venting minimizes the formation of bubbles in the fluid specimen. As noted above, bubbles reduce the volume of liquid in the testing cartridge and can affect test results. The ability of the locator adaptor to provide small droplets and to vent air adjacent the entry port substantially reduces the probability of overfill or spillage that might otherwise be achieved with a large diameter cylindrical or conical structure for feeding a liquid specimen into the testing cartridge, such as the conventional Luer tip of a prior art syringe.

The adaptor can be used by first drawing a specimen of blood or other bodily fluid with a syringe assembly substantially in a conventional manner. For example, the Luer tip of the syringe body or the blunt plastic cannula mounted to the Luer tip may be placed in communication with the fitting of an IV access system or fluid collection set. Alternatively, a conventional needle assembly may be mounted to the Luer tip of the syringe body and the distal tip of the needle cannula can be inserted into a blood vessel of the patient or other source of bodily fluid to obtain the required specimen. With either of these approaches, fluid is drawn through the passage of the Luer tip and into the fluid receiving chamber of the syringe body by pulling the plunger of the syringe assembly in a proximal direction. Most point-of-care testing cartridges require between 40 µl and 125 µl to complete a test. Hence, the plunger of the syringe assembly is moved proximally to obtain a volume of fluid slightly in excess of the amount required by the particular testing cartridge that will be employed.

The distal end of the syringe then is placed in communication with the proximal end of the locator adaptor. This may involve mere slidable insertion of a Luer tip into the conically tapered portions of the passage at the proximal end of the locator adaptor to achieve a fluid-tight frictional engagement. Alternatively, lugs at the proximal end of the locator adaptor can be threaded into engagement with a conventional Luer collar to achieve both a fluid-tight communication and a secure mechanical engagement. A protective cap that may be mounted over the distal end of the locator adaptor then may be removed.

The point-of-care testing cartridge then is removed from the manufacturer's package. Many manufacturers of testing cartridges provide a cover for the inlet port that is hinged into a covering disposition over the inlet port both prior to and after deposition of blood sample into the testing cartridge. Thus, a cover, if present on the testing cartridge, must be rotated away from the inlet port of the testing cartridge. The nose at the distal end of the locator adaptor then is guided into the entry port of the testing cartridge. This guiding of the nose is facilitated by the relatively small external diameter of the nose and by the stepped configuration of the fins disposed proximally of the nose. The nose is inserted into the entry port of the testing cartridge until distal ends of the fins engage portions of the top wall of the testing cartridge adjacent the entry port. The channels between the fins define vents that extend from the interior of the reservoir and entry port to the ambient surroundings. Additionally, the distal ends of the fins perform a depth control function to prevent over insertion of the nose into the entry port. The plunger of the syringe assembly then is moved distally to urge a selected volume of the specimen from the fluid receiving chamber of the syringe body, through the adaptor and into the testing cartridge. The syringe assembly and the locator adaptor then are removed from the testing cartridge and are discarded in a conventional safe manner. The cap of the testing cartridge then is sealingly engaged over the entry port of the testing cartridge, and the testing cartridge is presented to a portable clinical analyzer substantially in the conventional manner. Alternatively the testing cartridge can be mounted to the portable clinical analyzer before depositing the specimen in the testing cartridge.

### DESCRIPTION OF THE DRAWINGS

FIG. 1 is a perspective view of a locator adaptor in accordance with the subject invention.
FIG. 2 is a cross-sectional view taken along line 2-2 in FIG. 1.
FIG. 3 is a perspective view of a syringe for use with the locator adaptor shown in FIGS. 1 and 2.
FIG. 4 is a perspective view of a testing cartridge for use with the adaptor.

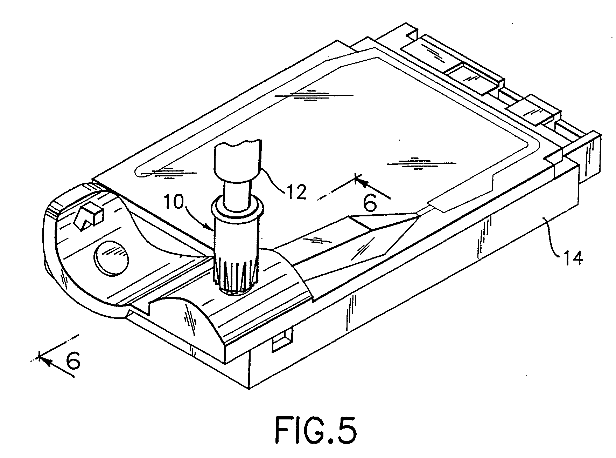

FIG. 5 is a perspective view of the syringe and locator adaptor engaged with the testing cartridge.

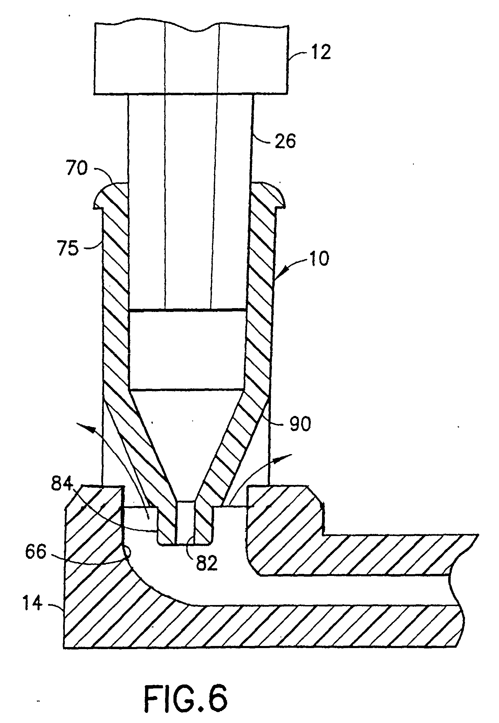

FIG. 6 is a cross-sectional view taken along line 6-6 in FIG. 5.

### DETAILED DESCRIPTION

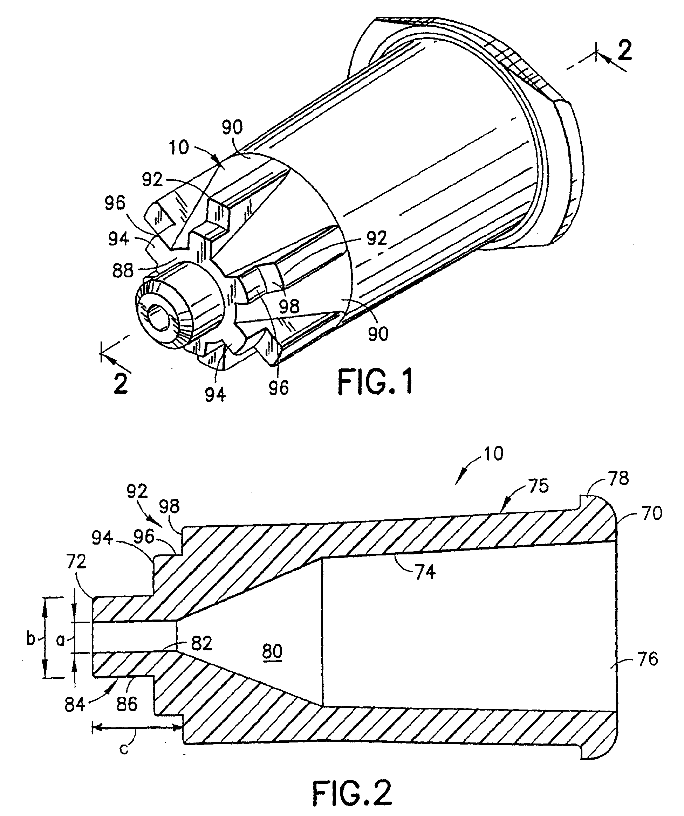

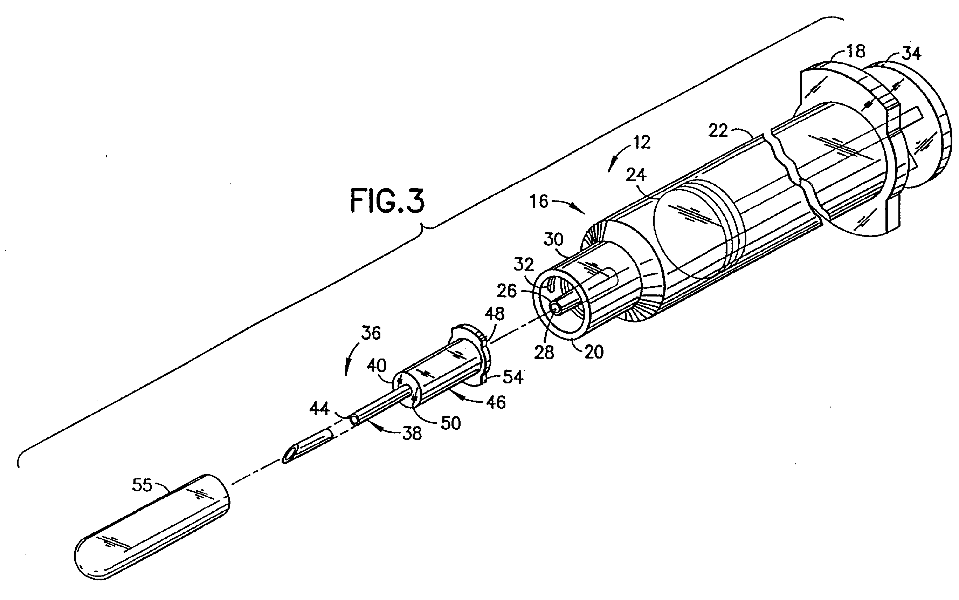

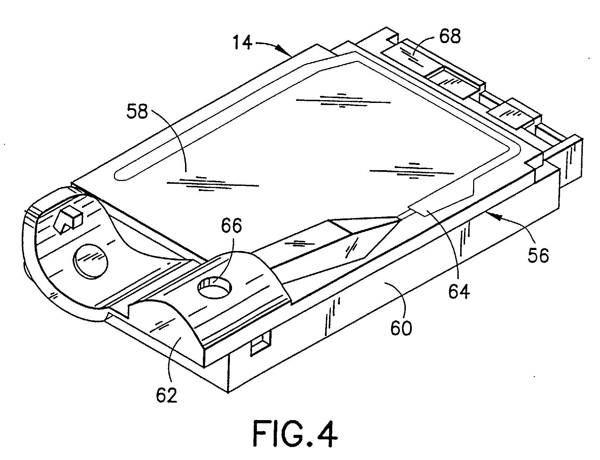

A locator adaptor in accordance with the subject invention is identified generally by the numeral **10** in FIGS. 1-4. Adaptor **10** is used with a syringe assembly **12,** as shown most clearly in FIG. 3, and with a point-of-care testing cartridge **14,** as shown most clearly in FIG. 4.

Syringe assembly **12,** as shown in FIG. 3, includes a syringe body **16** having a proximal end **18** and a distal end **20.** A barrel **22** extends distally from proximal end **18** and defines a cylindrical fluid receiving chamber **24** that is widely open at proximal end **18.** A frustoconically tapered tip **26** extends from barrel **22** to distal end **20** of syringe body **16.** Tip **26** is provided with a narrow cylindrical passage **28** that communicates with fluid receiving chamber **24** of barrel **22.** An optional Luer collar **30** projects distally from barrel **22** and concentrically surrounds tip **26.** Luer collar **30** is provided with an internal array of threads **32.** Syringe assembly **12** further includes a plunger **34** slidably disposed in fluid receiving chamber **24** and in fluid-tight engagement with the cylindrical walls of chamber **22.** Plunger **34** can be moved alternately in proximal or distal directions for urging fluid through passage **28** in tip **26** and into or out of fluid receiving chamber **24.**

Syringe assembly **12** optionally includes a needle assembly **36.** Needle assembly **36** includes a metallic needle cannula **38** having a proximal end **40,** a sharply pointed distal end **42** and a lumen **44** extending between the ends. Needle assembly **36** further includes a hub **46** that has a proximal end **48,** a distal end **50** and a passage extending therebetween. Distal end **50** of hub **46** is securely mounted to proximal end **40** of needle cannula **38** such that the passage through hub **46** communicates with lumen **44** through needle cannula **38.** The passage of hub **46** defines a taper that substantially matches tapered distal tip **26** on syringe body **16.** Thus, tapered tip **26** of syringe body **16** can be placed in fluid-tight frictional engagement with the passage in proximal end **48** of hub **46.** Proximal end **48** of hub **46** is further characterized by a pair of diametrically opposite lugs **54** that are dimensioned and configured for engagement with threads **32** of Luer collar **30.** Thus, lumen **44** through needle cannula **38** can be placed in communication with passage **28** in tip **26** and with fluid receiving chamber **24** of syringe body **16.** Needle assembly **36** further includes a protective cap **55** removably engaged over needle cannula **38.**

Point-of-care testing cartridge **14** is shown in FIG. 4 and may be of any of several prior art designs, including those manufactured by i-STAT Corporation, Diametrics Medical, Inc., AVL Scientific Corporation or any other such testing cartridges that are available or become available. One such testing cartridge is disclosed in U.S. Patent No. 5,638,828, the disclosure of which is incorporated herein by reference.

Testing cartridge **14** includes a generally rectangular body **56** with a top wall **58** that has a length of approximately 1.5-2.0 inches and a width of about 1.0 inches. Body **56** further has side walls **60** and end walls **62** that define a thickness for body **56** of about 0.25 inches. A fluid reservoir **64** is formed inside body **56** of cartridge **14** and has a volume in the range of 65 µl and 110 µl. Body **56** further includes an entry port **66** that extends through top wall **64** and communicates with reservoir **64.** Entry port **66** is slightly tapered from a relatively large diameter portion externally on housing **56** to a relatively smaller cross-section closer to reservoir **58.** Testing cartridge **14** further includes contact pads and sensors **68** that can be placed in communication with a portable clinical analyzer for performing various point-of-care diagnostic tests on the sample of blood in the reservoir **64** and for providing various readout data that can be used by a health care technician at the point-of-care and/or at a remote location.

Adaptor **10** includes a proximal end **70,** a distal end **72** and a passage **74** extending between ends **70** and **72.** Portions of adaptor **10** adjacent proximal end **70** define a Luer fitting **75** for mating with distal end of syringe assembly **12** as explained herein. More particularly, a portion of passage **74** in Luer fitting **75** and adjacent proximal end **70** is widely open and defines a Luer receptacle **76** with a slight conical taper configured for fluid tight engagement over Luer tip **26** of syringe assembly **12.** Additionally, external portions of Luer fitting **75** of adaptor **10** adjacent proximal end **70** include a pair of diametrically opposite lugs **78** that are configured for mating with internal threads **32** of Luer collar **30** on syringe assembly **12.** Of course, lugs **78** are not required if adaptor **10** is dedicated for use with a syringe that has no Luer collar. Passage **74** includes a sharply tapered section **80** distally of Luer receptacle **76.** Passage **74** further includes a small diameter cylindrical outlet passage **82** that extends from the distal end of sharply tapered section **80** of passage **74** to distal end **72** of adaptor **10.** Outlet passage **82** defines an inside diameter **a** that is sufficiently small to produce small droplets of the fluid specimen that will be delivered from syringe assembly **12.** The small droplets improve the ease of fill of testing cartridge **14** and substantially avoid spillage or overfill that often occurs in efforts to deliver large drops of fluid into the small entry port **66** of testing cartridge **14.** In a preferred embodiment, inside diameter **a** of outlet passage **82** is approximately equal to 0.03-0.04 inches.

Adaptor **10** further includes a nose **84** adjacent distal end **72.** Nose **84** includes a cylindrical outer surface **86** that is substantially concentric with outlet passage **82.** Cylindrical outer surface **86** defines an outside diameter **b** that is smaller than the inside diameter defined by entry port **66** of testing cartridge **14.** In a preferred embodiment, outside diameter **b** is approximately equal to 0.065-0.075 inches. Nose **84** further defines an axial length **c** which is approximately equal to outside diameter **b** in the illustrated embodiment. The length **c** of nose **84** is sufficiently long to facilitate visual guiding of nose **84** into entry port **66** of testing cartridge **14.** However, nose **84** is sufficiently short to ensure that outlet passage **82** is not blocked by surfaces of testing cartridge **14** in proximity to entry port **66.**

Exterior regions of locator adaptor **10** include a small annular shoulder **98** extending radially outwardly from cylindrical outer surface **96** of nose **84.** Annular shoulder **98** defines an outside diameter less than the inside diameter of entry port **66** of testing cartridge **14.**

Adaptor **10** further includes a conically generated surface **90** that extends outwardly from shoulder **88** to Luer fitting **75** at proximal end **70** of adaptor **10.** Conical surface **90** is interrupted by a plurality of radially aligned fins **92** that project radially outwardly from conical surface **90** and that extend from shoulder **88** to Luer fitting **75.** Each fin **92** is of stepped configuration and includes a distal face **94** aligned in substantially coplanar relationship with annular shoulder **88.** Each fin **92** then further includes an outer circumferential face **96** extending proximally from distal face **94.** Outer faces **96** of fins **92** are arranged to define portions of a cylindrical surface. Each fin **92** further includes an offset face **98** which is spaced proximally from distal face **94.** Thus, as shown most clearly in FIG. 1, portions of locator adaptor **10** disposed distally of Luer fitting **75** define a conical or stepped taper that facilitates visual alignment of nose **84** with entry port **66** of testing cartridge **14.** Conically generated surface regions **90** between fins **92** function as channels to facilitate venting when portions of a blood specimen are urged into entry port **66** of testing cartridge **14.**

Syringe assembly **12** is used in a conventional manner to draw a sample of fluid from a patient. More particularly, needle assembly **36** can be mounted to Luer tip **26** of syringe body **16,** and needle cannula **38** of needle assembly **36** can be inserted into a blood vessel of a patient or other source of bodily fluid for drawing a sample of blood or other such fluid. Alternatively, a blunt plastic cannula or other plastic Luer fitting can be mounted to Luer tip **26,** and the distal end of the blunt plastic cannula or other fitting can be urged through the septum that seals a fitting of a fluid collection set. Still further, syringe assembly **12** can be connected directly to an arterial or venous line that had already been placed in communication with a patient. With any of these optional approaches, plunger **34** is moved proximally after accessing the supply of fluid. Proximal movement of plunger **34** draws fluid into fluid receiving chamber **24** of syringe barrel **22.** The volume of fluid drawn into fluid receiving chamber **24** is in excess of the volume of fluid required for testing cartridge **14,** which typically is in the range of 40µl - 125µl. Needle assembly **36** or the blunt plastic cannula, if used, then is removed from syringe body **16** substantially in a conventional manner and is disposed of in a sharps receptacle.

Luer tip **26** of syringe assembly **12** then is placed in communication with Luer receptacle **76** of passage **74** at proximal end of locator adaptor **10.** This may involve threadedly engaging projection **78** at proximal end **70** of adaptor **10** with internal threads **32** of Luer collar **30** if such a Luer collar is provided on the syringe.

Nose **84** of adaptor **10** then is guided into entry port **66** of testing cartridge **14.** The stepped and tapered configuration of portions of adaptor **10** distally of Luer fitting **75** facilitate location and alignment of nose **84** with entry port **66.** Shoulder **88** of adaptor **10** defines an outside diameter that is less than the inside diameter of entry port **66.** Accordingly, shoulder **88** does not create an air lock within testing cartridge **14.** Rather, sections of adaptor **10** between fins **92** define vent channels that facilitate and enable escape of air from testing cartridge **14.**

The use of testing cartridge **14** proceeds merely by urging plunger **34** distally in syringe body **16.** Movement of plunger **34** causes fluid in fluid receiving chamber **24** to be urged through Luer tip **26** of syringe body **16,** through passage **96** of adaptor **10** and into reservoir **64** of testing cartridge **14.** The relatively small inside diameter **a** of outlet passage **82** of adaptor **10** produce very small droplets of the fluid that can be deposited easily into entry port **66** of testing cartridge **14.** After a sufficient volume of fluid has been deposited into testing cartridge **14,** syringe assembly **12** and adaptor **10** can be separated from testing cartridge **14** and disposed of in an appropriate and safe manner. The cover of testing cartridge **14** then can be rotated into sealing engagement over entry port **66,** and testing cartridge **14** then can be presented to a portable clinical analyzer.

## Claims

1. A locator adaptor for use with a syringe and a testing cartridge, said syringe having a Luer tip at one end and said testing cartridge having an entry port with an inside diameter, said locator adaptor comprising opposite proximal and distal ends and a passage extending between said ends, portions of said locator adaptor adjacent said proximal end defining a Luer fitting configured for mating with said Luer tip of said syringe, portions of said locator adaptor adjacent said distal end defining a nose cross-sectionally smaller than said entry port of said testing cartridge, a plurality of radially aligned fins between said nose and said Luer fitting of said locator adaptor, distal portions of said locator adaptor between said fins being cross-sectionally smaller than said inside diameter of said entry port for defining vent channels that enable venting of air from said testing cartridge when said nose is disposed in said entry port.

2. The locator adaptor of Claim 1, wherein said adaptor is unitarily molded from a plastic material.

3. The locator adaptor of Claim 1, wherein said nose includes a cylindrical outer surface.

4. The locator adaptor of Claim 1, further comprising an annular shoulder adjacent said nose, said shoulder being aligned orthogonal to said nose and defining a maximum outside diameter less than said inside diameter of said entry port.

5. The locator adaptor of Claim 3, wherein said fins each include a distal face, said distal faces of said fins defining a plane aligned substantially orthogonal to said nose, said distal faces of said fins defining outside cross-sectional dimensions greater than said inside diameter of said entry port for limiting depth of insertion of said nose into said entry port.

6. The locator adaptor of Claim 5, wherein radially outer and distal portions of said fins are stepped to facilitate alignment and location of said nose with said entry port.

7. The locator adaptor of Claim 6, wherein portions of said locator adaptor between said fins are conically tapered to facilitate visual alignment of said nose with said entry port.

8. The locator adaptor of Claim 1, wherein said proximal end of said locator adaptor includes a pair of diametrically opposite projections for threaded engagement with a Luer collar of said syringe.

9. The locator adaptor of Claim 1, wherein portions of said passage in said nose are substantially cylindrical and define an inside diameter of between 0.03 inches and 0.04 inches.

10. The locator adaptor of Claim 9, wherein said nose includes an outer cylindrical surface defining an outside diameter of between approximately 0.065 inches and 0.075 inches.
